# EUROPÄISCHE PATENTANMELDUNG

(11) **EP 1 136 466 A2**
(43) Veröffentlichungstag der Anmeldung: **26.09.2001**
(21) Anmeldenummer: 01102629.1
(22) Anmeldetag: 07.02.2001
(51) Int. Cl.: C07B 55/00, C07C 231/16, C07C 233/47

(54) **Verfahren zur Racemisierung von N-Acylaminosäuren mittels Übergangsmetallkatalysatoren**

(30) Priorität: 18.03.2000 DE 10013599
(71) Anmelder: Aventis Research & Technologies GmbH & Co KG, 65926 Frankfurt am Main (DE)
(72) Erfinder: Riermeier, Thomas, Dr., 65439 Flörsheim (DE); Beller, Matthias, Prof. Dr., 18119 Rostock (DE); Schichl, Daniel, 85784 Garching (DE); Hateley, Martin, Dr., Nr Stourbridge, Worcestershire DY9 0LX (GB)
(74) Vertreter: Ackermann, Joachim, Dr.

(57) **Zusammenfassung**

Gegenstand der vorliegenden Erfindung ist ein Verfahren zur Racemisierung von *N*-Acylaminosäuren der allgemeinen Formel (I), 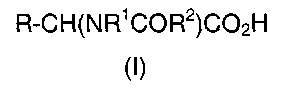 bei dem in einem Lösungsmittel ein enantiomerenangereichertes Gemisch oder eine enantiomerenreine Verbindung der Formel (I), in Gegenwart eines Übergangsmetallsalzes, Übergangsmetallkomplexes oder Übergangsmetallkomplexsalzes oder eines Gemisches davon, enthaltend mindestens ein Element aus der Gruppe Fe, Ru, Os, Co, Rh, Ir, Ni, Pd, Pt, bevorzugt bei einer Temperatur von 10 - 150 °C umgesetzt wird.

## Beschreibung

Die Erfindung betrifft ein Verfahren zur Racemisierung enantiomerenreiner *N*-Acylaminosäuren mit Übergangsmetallkatalysatoren zur Herstellung racemischer *N*-Acylaminosäuren der Formel (I).

Aminosäuren und deren Derivate gehören unbestritten zu den wichtigsten organischen Verbindungsklassen. Neben der biochemischen Bedeutung sind Aminosäurederivate als Wirkstoffzwischenprodukte, Agrochemikalien, Nahrungsmittelzusatzstoffe sowie industrielle Feinchemikalien von großem wirtschaftlichem Interesse. Trotz der Entwicklung von enantioselektiven Herstellverfahren beispielsweise nach Schöllkopf (U. Schöllkopf, *Top. Curr. Chem.* **1983,** *109,* 65), Seebach (D. Seebach, A. R. Sting, M. Hoffmann, *Angew. Chem.* **1996,** *108,* 2880), Evans (D. A. Evans, A. E. Weber, *J. Am. Chem. Soc.* **1987,** *109,* 7151; D. A. Evans, T. C. Britton, J. A. Ellman, R. L. Dorow, *J. Am. Chem. Soc.* **1990,** *112,* 4011), Williams (R. M. Williams, P. J. Sinclair, D. Zhai, D. Chen, *J. Am. Chem. Soc.* **1988,** *110,* 1547; R. M. Williams, , *Vol. 7*, Pergamon Press 1989) sowie neuesten Methodenentwicklungen (N. A. Petasis, I. A. Zavialov, *J. Am. Chem. Soc.* **1997,** *119,* 445-446; Y. S. Park, P. Beak, *J*. Org. *Chem*. **1997,** *62*, 1574-1575; A. G. Myers, J. L. Gleason, T. Yoon, D. W. Kung, *J. Am*. *Chem. Soc.* **1997,** *119,* 656-673; B. M. Trost, *Science* **1991,** *254*, 1471-1477) werden industriell zur Herstellung von nicht-natürlichen Aminosäuren im allgemeinen "klassische Methoden" wie die Strecker-Synthese mit nachfolgender *N*-*Ac*ylierung und Racematspaltung angewandt. Der Vorteil derartiger Drei-Stufenverfahren liegt insbesondere im Preis und der Verfügbarkeit der Ausgangsmaterialien sowie der Praktikabilität der einzelnen Reaktionsschritte.

Enzymatische Racematspaltungen verschiedener *N*-Acylaminosäuren gelingen mit einer Vielzahl funktionalisierter *N*-Acylaminosäuren in Gegenwart von Acylasen. Zusammenstellungen findet man beispielsweise in J. P. Greenstein, M. Winitz in "*Chemistry of the Amino Acids"* John Wiley & Sons, London, **1961,** Vol 1, 715-760; H. Keith, J. Dahmer, G. M. Whitesides, *J. Am. Chem*. *Soc,* **1989,** 111, 6354-6364.. Generell wird bei den Racematspaltungsverfahren das unerwünschte verbleibende Enantiomer der *N*-Acylaminosäure nach der Racematspaltung wieder racemisiert, so daß in einem Mehrstufenprozess nahezu quantitative Ausbeuten an der gewünschten enantiomerenreinen Aminosäure erzielt werden. Als Racemisierungskatalysatoren für *N*-Acylaminosäuren werden in der Literatur bis dato Säurekatalysatoren bei höheren Temperaturen eingesetzt (DE 3334849). Aufgrund der drastischen Reaktionsbedingungen und der unspezifischen Reaktivität der Säurekatalysatoren kommt es bei empfindlichen funktionalisierten *N*-Acylaminosäuren zu Nebenreaktionen, die die Gesamtausbeute vermindern und die Produktisolierung signifikant erschweren. Alternativ dazu kann mit stöchiometrischen Mengen an Keten racemisiert werden (DE 2740380). Wegen der hohen Toxizität und der unpraktikablen Handhabbarkeit von Keten ist diese Methode allerdings kaum von praktischen Nutzen.
Vor diesem Hintergrund ist die Entwicklung neuer schonender Racemisierungskatalysatoren von großem Interesse. Aufgabe der vorliegenden Erfindung war es folglich ein Verfahren zur Racemisierung von *N*-Acylaminosäuren bereitzustellen, welches unter milden Bedingungen abläuft.

Überraschend wurde gefunden, daß enantiomerenreine oder enantiomerenangereicherte *N-Ac*ylaminosäuren am stereogenen Zentrum (α-C-Zentrum) in Gegenwart von Übergangsmetallkatalysatoren unter milden Reaktionsbedingungen racemisiert werden können.

Gegenstand der vorliegenden Erfindung ist ein Verfahren zur Racemisierung von N-*Ac*ylaminosäuren der Formel (I),

R-CH(NR¹COR²)CO₂H (I)

worin R, R¹ und R² unabhängig voneinander für einen Wasserstoff und/oder Alkyl-, Alkenyl, Alkinyl, Aryl- und/oder Heteroarylrest steht, wobei Alkyl eine aliphatische Kohlenstoffgruppe mit 1 bis 18 C-Atomen, die linear, verzweigt und/oder auch cyclisch sein kann, und Alkenyl bzw. Alkinyl eine ein- oder mehrfach ungesättigte aliphatische Gruppe mit 2 bis 18 C-Atomen, die verzweigt oder nichtverzweigt sein können, bedeutet und Aryl einen 4 bis 14 C-Atome enthaltenden fünf-, sechs- oder siebengliedrigen aromatischen Ring, wobei dieser Ring anneliert sein kann und 0 bis 3 Heteroatome wie N, O, S enthalten kann, bedeutet, dabei können sowohl die Alkyl- als auch die Arylgruppe gegebenenfalls bis zu sechs weitere Substituenten tragen, die unabhängig voneinander Wasserstoff, Alkyl, O-Alkyl, OCO-Alkyl, O-Aryl, Aryl, Fluor, Chlor, Brom, lod, OH, CF₃, NO₂, NO, SiAlkyl₃, CN, COOH, CHO, SO₃H, NH₂, NH-Alkyl, N-Alkyl₂, PO-Alkyl₂, SO₂-Alkyl, SO-Alkyl, CF₃, NHCO-Alkyl, CONH2, CO-Alkyl, COO-Alkyl, NHCOH, NHCOO-Alkyl, CO-Aryl, COO-Aryl, POAryl₂, PO₃H₂, PO(O-Alkyl)₂, SO₃-Alkyl bedeuten, wobei Alkyl und Aryl die oben genannte Bedeutung haben,
das dadurch gekennzeichnet ist, daß in einem Lösungsmittel ein enantiomerenangereichertes Gemisch oder eine enantiomerenreine Verbindung der Formel (I), in Gegenwart eines Übergangsmetallsalzes, Übergangsmetallkomplexes oder Übergangsmetallkomplexsalzes oder eines Gemisches davon, enthaltend mindestens ein Element aus der Gruppe Fe, Ru, Os, Co, Rh, Ir, Ni, Pd, Pt, bevorzugt bei einer Temperatur von 10- 150 °C umgesetzt wird.

Durch Kombination der hier beschriebenen katalytischen Racemisierung mit einer Kristallisation eines Enantiomers in Gegenwart eines chiralen Racematspaltungsreagenz bzw. mit einer acylasekatalysierten Deacylierung sind erstmals auch dynamisch-kinetische Racematspaltungen von racemischen *N*-Acylaminosäuren durchführbar.

Das erfindungsgemäße Verfahren ist darüber hinaus sehr ökonomisch und sowohl einfach als auch schnell durchführbar. Nach Beendigung der Reaktion wird das racemisierte Produkt durch einfache Maßnahmen, z. B. durch Extraktion oder Kristallisation gereinigt oder es wird als Rohprodukt direkt einer Racematspaltung, wie z. B. Umsetzung mit einer Acylase, Kristallisation mit einem chiralen Racematspaltungsreagenz, wie einer chiralen Basen oder direkte Kristallisation, unterworfen. Gegebenenfalls kann ein solches Verfahren auch *in situ* durchgeführt werden, so daß eine dynamische kinetische Racematspaltung resultiert.

Ferner ist Mithilfe der gefundenen Methode eine asymmetrische Transformationen von *N-Ac*ylaminosären, wie sie beispielsweise in *Bull. Chem. Soc. Jpn.* **1981,** *54,* 3286 beschrieben, unter deutlich milderen und schonenderen Bedingungen durchführbar.

### Bevorzugt steht in den Verbindungen der Formel (I)

R, R¹ und R² unabhängig voneinander für Wasserstoff, (C₄-C₁₄)-Aryl, (C₁-C₁₈)-Alkyl, (C₂-C₁₈)-Alkenyl bzw. (C₃-C₁₈)-Alkinyl, CₙH₂ₙ-Cycloalkyl mit n = 3-18, die verzweigt oder nichtverzweigt sein können und die substituiert sein können mit 1-6 Substituenten, die unabhängig voneinander Wasserstoff, Alkyl, O-Alkyl, O-Aryl, Aryl, Fluor, Chlor, Brom, lod, OH, NO₂, CN, COOH, CHO, NH₂, NH-Alkyl, N-Alkyl₂, PO-Alkyl₂, SO₂-Alkyl, CF₃, NHCO-Alkyl, CONH₂, CO-Alkyl, NHCOH, NHCOO-Alkyl, CO-Aryl, COO-Aryl, POAryl₂, PO₃H₂, PO(O-Alkyl)₂ bedeuten, und Aryl einen 4 bis zu 14 C-Atome enthaltenden fünf-, sechs- oder siebengliedrigen aromatischen Ring, wobei dieser Ring anneliert sein kann und 0 bis 3 Heteroatome wie N, O, S enthalten kann, bedeutet.

### Besonders bevorzugt steht in den Verbindungen der Formel (I)

R, R¹ und R² unabhängig voneinander für Wasserstoff (C₅-C₁₂)-Aryl, (C₁-C₁₆)-Alkyl, (C₂-C₁₆)-Alkenyl bzw. (C₃-C₁₆)-Alkinyl, CₙH₂ₙ-Cycloalkyl mit n = 3-16, die verzweigt oder nichtverzweigt sein können und die substituiert sein können mit 1-4 Substituenten, die unabhängig voneinander Wasserstoff, Alkyl, O-Alkyl, O-Aryl, Aryl, Fluor, Chlor, Brom, lod, OH, NO₂, CN, COOH, CHO, NH2, NH-Alkyl, N-Alkyl₂, CF₃, NHCO-Alkyl, CONH₂, CO-Alkyl, NHCOH, NHCOO-Alkyl, CO-Aryl, COO-Aryl, bedeuten, und Aryl einen 4 bis zu 12 C-Atome enthaltenden fünf-, sechs- oder siebengliedrigen aromatischen Ring, wobei dieser Ring anneliert sein kann und 0 bis 3 Heteroatome wie N, O, S enthalten kann, bedeutet.

### Ganz besonders bevorzugt steht in den Verbindungen der Formel (I)

R, R¹ und R² unabhängig voneinander für Wasserstoff (C₅-C₁₀)-Aryl, (C1-C14)-Alkyl, (C₂-C₁₄)-Alkenyl bzw. (C₃-C₁₄)-Alkinyl, CₙH₂ₙ-Cycloalkyl mit n = 3-14, die verzweigt oder nichtverzweigt sein können und die substituiert sein können mit 1-3 Substituenten, die unabhängig voneinander Wasserstoff, Alkyl O-Alkyl, O-Aryl, Aryl, Fluor, Chlor, Brom, lod, OH, NO₂, CN, COOH, CHO, NH2, NH-Alkyl, N-Alkyl₂, CF₃, NHCO-Alkyl, CONH₂, CO-Alkyl, CO-Aryl, COO-Aryl, bedeuten, und Aryl einen 4 bis zu 10 C-Atome enthaltenden fünf-, sechs- oder siebengliedrigen aromatischen Ring, wobei dieser Ring anneliert sein kann und 0 bis 3 Heteroatome wie N, O, S enthalten kann, bedeutet.

Beispiele für *N*-Acylaminosäuren, die nach dem erfindungsgemäßen Verfahren racemisiert werden können, ohne dieses darauf zu beschränken, sind: *N*-*Ac*ylvalin, *N-Ac*ylvinylglycin, *N-Ac*ylphenylalanin, *N-Ac*ylmethionin, *N*-*Ac*ylphenylglycin, *N-Acyl*-4-hydroxyphenylglycin, *N-Ac*yl-3-pyridylalanin, *N-Ac*yltryptophan, *N-Ac*yllysin, *N-Ac*yl-*tert*-leucin, *N-Ac*ylprolin, *N-Ac*ylcyclohexylglycin, *N-Ac*ylserin, *N-Ac*yl-phosphinothricin. Dabei steht Acyl bevorzugt für Acetyl, Benzoyl, Phenylacetyl, Methoxyacetyl, Chloracetyl, und Propionyl.

Bevorzugt verwendet man als Racemisierungskatalysatoren Salze, Komplexe bzw. Salze der Komplexe der Übergangsmetalle Fe, Ru, Ir, Rh, Co und Pd.

Besonders bevorzugt sind als Racemisierungskatalysatoren Verbindungen der Elemente Ru, Ir, Rh, Pd. Beispiele für Komplexe dieser Elemente die eingesetzt werden können sind: [Rh(cod)Cl]₂, (CO)₂Rhacac, Rh(cod)acac, Rh(PPh₃)₃Cl, Ir(PPh₃)₃Cl, [Ru(*p*-cymene)Cl]₂, Rh(cod)₂BF₄, [Ru(CO)₂CH₃CO₂]ₙ, Ru(CO)(H₂)(PPh₃)₃, Ru(cod)Cl₂, lr(cod)₂BF₄, IrCl(CO)(PPh₃)₂, Ir(CO)₂acac, Ru(acac)₃, Pd(PPh₃)₄, PdCl₂(CH₃CN)₂, Pd(OAc)₂, PdCl₂, Li₂PdCl₄, Pd2(dba)3.

In der Regel ist es vorteilhaft, wenn der Racemisierungskatalysator in homogener Form vorliegt. Jedoch können heterogene bzw. heterogenisierte Metallkomplexe insbesondere bei Reaktionstemperaturen > 100°C vorteilhaft eingesetzt werden.

Einzusetzende heterogene Katalysatoren sind beispielsweise Rhodium, Iridium bzw. Ruthenium auf Trägern wie Aktivkohle, Aluminiumoxid, Kieselgel und Titandioxid.

Zur Stabilisierung aber auch zur Aktivitätserhöhung der Katalysatoren ist der Zusatz stickstoffhaltigen und/oder phosphorhaltigen Liganden, wie z. B. von Amin- und/oder Phosphanliganden vorteilhaft. Typischerweise eingesetzte Phosphanliganden sind Triphenylphosphan, Tricyclohexylphosphan, Tri-tert.butyl-phosphan, TPPTS (Tris(3-sulfonatophenyl)phosphan), Tri-n-butylphosphan, Bisdiphenylphosphinopropan, Trio-tolylphosphan, Trimethylphosphat, Diphenyl-2-pydridylphosphan, Bis(dicyclohexylphosphino)butan. Als Aminliganden können beispielsweise verwendet werden Triethylamin, Pyridin, Bipyridin, Tetramethylethylendiamin, Triazacyclononan, Piperazin.

Die Reaktion wird zweckmäßigerweise in einem Solvenz durchgeführt. Als Lösungsmittel können organische Lösungsmittel oder Wasser verwendet werden. Es kann vorteilhaft sein, Gemische an Lösungsmitteln zu verwenden. Als Solvenz eignen sich z. B. Acetonitril, Butyronitril, Essigester, Dimethoxyethan, Aceton, THF, Dioxan, Hexan, tert.-Butylmethylether und tert.-Butanol, Toluol, DMSO, Chloroform und beziehungsweise Mischungen davon. Der Anteil an N-Acylaminosäure in der Lösung liegt vorzugsweise bei 5 - 50 %.

Die Herstellung der N-Acylaminosäuren erfolgt zweckmäßigerweise aus der Aminosäure nach bekannten Methoden der Acylierung.

Nach dem erfindungsgemäßen Verfahren wird der Racemisierungskatalysator bevorzugt in katalytischen Mengen bezüglich der N-Acylaminosäure eingesetzt. Generell werden zwischen 0.2 und 0.0001 Equivalente bezogen auf N-Acylaminosäure, bevorzugt 0.15 bis 0.001 und besonders bevorzugt 0.1 bis 0.001 Equivalente verwendet.

Durch die nachfolgenden Beispiele soll die vorliegende Erfindung näher erläutert werden, ohne diese darauf zu beschränken.

Beispiele:
1) Racemisierung von (*S*)-*N*-Acetylvalin
2) Racemisierung von (*S*)-*N*-Acetylprolin
3-5) Racemisierung von (*S*)-*N*-Acetylphenylalanin
6-12) Racemsierung von (*S*)-*N*-Acetylphenylalanin mit verschiedenen Katalysatorsystemen

Alle isolierten Produkte bzw. Rohproduktgemische wurden durch ¹H-NMR- und Massen-Spektren bzw per HPLC identifiziert.
Die optische Reinheit der Produkte wurde durch HPLC, z. B an Chiralpak AD 250 X 4.6 (Daicel) bestimmt.

### Beispiel 1:

Eine Lösung aus (*S*)-*N*-Acetylvalin (83.7 mg, 0.483 mmol), Tricyclohexylphosphan (67.7 mg, 0.242 mmol), [Rh(cod)Cl]₂, (11.9 mg, 0.0242 mmol) in 10 ml Acetonitril wird für 48 Stunden auf 60 °C erhitzt. Anschließend entfernt man das Lösungsmittel, gibt 10 ml einer wässrigen 1 n NaOH-Lösung zu und extrahiert mit Ethylacetat (3 x 10 ml). Die wässrige Phase wird mit Schwefelsäure angesäuert und *N*-Acetylvalin mit Ethylacetat (3 x 20 ml) extrahiert. Die organische Phase wird über MgSO₄ getrocknet. Nach Entfernen des Lösungsmittels am Vakuum erhält man *N*-Acetylvalin mit einem Enantiomerenüberschuß von 8.0 % (mittels GC, ee-Wert des Methylesterderivates).

### Beispiel 2:

Eine Lösung aus (S)-*N*-Acetylprolin (75.9 mg, 0.483 mmol), Tricyclohexylphosphan (67.7 mg, 0.242 mmol), [Rh(cod)Cl]₂, (11.9 mg, 0.0242 mmol) in 10 ml Acetonitril wird für 48 Stunden auf 60 °C erhitzt. Nach Aufarbeitung (siehe Beispiel 1) erhält man N-Acetylprolin mit einem Enantiomerenüberschuß von 1.0 % (mittels GC, ee-Wert des Methylesterderivates).

### Beispiel 3:

Eine Lösung aus (*R*)-*N*-Acetylphenylalanin (100 mg, 0.483 mmol), Triphenylphosphan (63.2 mg, 0.242 mmol), [Rh(cod)Cl]₂, (11.9 mg, 0.0242 mmol) in 10 ml Acetonitril wird für 48 Stunden auf 60 °C erhitzt. Nach Aufarbeitung (siehe Beispiel 1) erhält man *N*-Acetylphenylalanin mit einem Enantiomerenüberschuß von 34.0 % (mittels GC, ee-Wert des Methylesterderivates).

### Beispiel 4:

Eine Lösung aus (*S*)-*N*-Acetylphenylalanin (100 mg, 0.483 mmol), Tricyclohexylphosphan (67.7 mg, 0.242 mmol), [Rh(cod)Cl]₂, (11.9 mg, 0.0242 mmol) in 10 ml Toluol wird für 48 Stunden auf 100 °C erhitzt. Nach Aufarbeitung (siehe Beispiel 1) erhält man *N*-Acetylphenylalanin mit einem Enantiomerenüberschuß von 5.4 % (mittels GC, ee-Wert des Methylesterderivates).

### Beispiel 5:

Eine Lösung aus (*S*)-*N*-Acetylphenylalanin (100 mg, 0.483 mmol), Tricyclohexylphosphan (67.7 mg, 0.242 mmol), [Rh(cod)Cl]₂, (11.9 mg, 0.0242 mmol), Tetrabutylammoniumbromid (15.6 mg, 0.048 mmol) in 10 ml Aceton wird für 48 Stunden auf 50 °C erhitzt. Nach Aufarbeitung (siehe Beispiel 1) erhält man *N*-Acetylphenylalanin mit einem Enantiomerenüberschuß von 17.0 % (mittels GC, ee-Wert des Methylesterderivates).

### Beispiel 6 - 12:

Eine Lösung aus (*S*)-*N*-Acetylphenylalanin (100 mg, 0.483 mmol), Tricyclohexylphosphan (67.7 mg, 0.242 mmol), 5 mol% Katalysator in 10 ml Acetonitril wird für 48 Stunden auf 60 °C erhitzt. Ergebnisse siehe Tabelle 1.

**Tabelle 1:**

| Racemisierung von (S)-N-Acetylphenylalanin mit verschiedenen Katalysatoren. | | | | | |
|---|---|---|---|---|---|
| Beispiel | Katalysator | Lösungs-mittel | Tem p [°C] | Reaktions-zeit [h] | ee-Wert |
| **6** | [Rh(cod)Cl]₂ | MeCN | 60 | 48 | 0.4-10.3 % |
| **7** | [Rh(nbd)Cl]₂ | MeCN | 60 | 48 | 18% |
| **8** | [Rh(cod)₂BF₄] | MeCN | 60 | 48 | 50 % |
| **9** | (CO)₂Rh(acac) | MeCN | 60 | 48 | 74 % |
| **10** | RhCl₃xH₂O | MeCN | 60 | 48 | 70 % |
| **11**^{[a]} | [Rh(cod)Cl]₂ | MeCN | 60 | 20 | 26% |
| **12** | [Rh(cod)Cl]₂ | MeCN | 60 | 20 | 30 % |
| [a] (*R*)-*N*-Acetylphenylalanin wurde verwendet. | | | | | |

### Vorbermerkungen zu den Beispielen 13-20

Folgende Umsetzungen wurden duchgeführt:
- 13-17): Racemisierung von (*S*)-*N*-Acetylphenylalanin mit verschiedenen Palladiumkatalysatoren
- 18): Racemisierung von (*S*)-*N*-Acetylleucin mit Pd(OAc)₂/PPh₃
- 19): Racemisierung von (*S*)-*N*-Acetylmethionin mit Pd(OAc)₂/PPh₃
- 20): Racemisierung von (*S*)-*N*-Acetylprolin mit Pd(OAc)₂/PPh₃

Alle isolierten Produkte bzw. Rohproduktgemische wurden durch 1H-NMR- und Massen-Spektren bzw. per HPLC identfiziert.
Die optische Reinheit der Produkte wurde durch HPLC, z.B. an Chiralpak AD 250 X 4.6 (Daicel) bestimmt.

### Beispiel 13:

Eine Lösung aus (*S*)-*N*-Acetylphenylalanin (100 mg, 0.483 mmol), Pd(PPh₃)₄ (5.6 mg, 0.00483 mmol) in 10 ml Acetonitril wird für 48 Stunden auf 60 °C erhitzt. Nach Aufarbeitung (siehe Beispiel 1) erhält man *N*-Acetylphenylalanin mit einem Enatiomerenüberschluß von 14.0 % (mittels GC, ee-Wert des Methylesterderivates).

### Beispiel 14:

Eine Lösung aus (*S*)-*N*-Acetylphenylalanin (100 mg, 0.483 mmol), Triphenylphosphan (6.3 mg, 0.02415 mmol), Pd(OAc)₂ (1.08 mg, 0.00483 mmol) in 10 ml Acetonitril wird für 48 Stunden auf 60 °C erhitzt. Nach Aufarbeitung (siehe Beispiel 1) erhält man *N*-Acetylphenylalanin mit einem Enatiomerenüberschluß von 8.0 % (mittels GC, ee-Wert des Methylesterderivates).

### Beispiel 15:

Eine Lösung aus (*S*)-*N*-Acetylphenylalanin (100 mg, 0.483 mmol), Triphenylphosphan (6.3 mg, 0.02415 mmol), Pd(OAc)₂ (1.08 mg, 0.00483 mmol) in 10 ml Acetonitril wird für 48 Stunden auf 80 °C erhitzt. Nach Aufarbeitung (siehe Beispiel 1) erhält man *N-Ac*etylphenylalanin mit einem Enatiomerenüberschluß von 3.0 % (mittels GC, ee-Wert des Methylesterderivates).

### Beispiel 16:

Eine Lösung aus (*S*)-*N*-Acetylphenylalanin (100 mg, 0.483 mmol), Tricyclohexylphosphan (6.8 mg, 0.02415 mmol), Pd(OAc)₂ (1.08 mg, 0.00483 mmol) in 10 ml Acetonitril wird für 48 Stunden auf 60 °C erhitzt. Nach Aufarbeitung (siehe Beispiel 1) erhält man *N*-Acetylphenylalanin mit einem Enatiomerenüberschluß von 76.0 % (mittels GC, ee-Wert des Methylesterderivates).

### Beispiel 17:

Eine Lösung aus (*S*)-*N*-Acetylphenylalanin (100 mg, 0.483 mmol), Triphenylphosphan (6.3 mg, 0.02415 mmol), Pd₂(dba)₃ (2.21 mg, 0.00483 mmol) in 10 ml Acetonitril wird für 48 Stunden auf 80 °C erhitzt. Nach Aufarbeitung (siehe Beispiel 1) erhält man *N*-Acetylphenylalanin mit einem Enatiomerenüberschluß von 1.0 % (mittels GC, ee-Wert des Methylesterderivates).

### Beispiel 18:

Eine Lösung aus (*S*)-*N*-Acetylleucin (83.7 mg, 0.483 mmol), Triphenylphosphan (6.3 mg, 0.02415 mmol), Pd(OAc)₂ (1.08 mg, 0.00483 mmol) in 10 ml Acetonitril wird für 48 Stunden auf 80 °C erhitzt. Nach Aufarbeitung (siehe Beispiel 1) erhält man (*S*)*-N*-Acetylleucin mit einem Enatiomerenüberschluß von 13.0 % (mittels GC, ee-Wert des Methylesterderivates).

### Beispiel 19:

Eine Lösung aus (*S*)-*N*-Acetylmethionin (92.4 mg, 0.483 mmol), Triphenylphosphan (15.8 mg, 0.0604 mmol), Pd(OAc)₂ (2.7 mg, 0.0121 mmol) in 10 ml Acetonitril wird für 48 Stunden auf 60 °C erhitzt. Nach Aufarbeitung (siehe Beispiel 1) erhält man (S)-*N*-Acetylmethionin mit einem Enatiomerenüberschluß von 9.0 % (mittels GC, ee-Wert des Methylesterderivates).

### Beispiel 20:

Eine Lösung aus (*S*)-*N*-Acetylprolin (75.9 mg, 0.483 mmol), Triphenylphosphan (6.3 mg, 0.02415 mmol), Pd(OAc)₂ (1.08 mg, 0.00483 mmol) in 10 ml Acetonitril wird für 48 Stunden auf 80 °C erhitzt. Nach Aufarbeitung (siehe Beispiel 1) erhält man *(S)-N*-Acetylprolin (mit einem Enatiomerenüberschluß von 10.0 % (mittels GC, ee-Wert des Methylesterderivates).

## Patentansprüche

1. Verfahren zur katalytischen Racemisierung von N-Acylaminosäuren der Formel (I),
R-CH(NR¹COR²)CO₂H (I)
worin R, R¹ und R² unabhängig voneinander für einen Wasserstoff und/oder Alkyl-, Alkenyl, Alkinyl, Aryl- und/oder Heteroarylrest steht, wobei Alkyl eine aliphatische Kohlenstoffgruppe mit 1 bis 18 C-Atomen, die linear, verzweigt und/oder auch cyclisch sein kann, und Alkenyl bzw. Alkinyl für eine ein- oder mehrfach ungesättigte aliphatische Gruppe mit 2 bis 18 C-Atomen steht, die verzweigt oder nichtverzweigt sein können, und Aryl einen 4 bis 14 C-Atome enthaltenden fünf-, sechs- oder siebengliedrigen aromatischen Ring darstellt, wobei dieser Ring anneliert sein kann und 0 bis 3 Heteroatome wie N, O, S enthalten kann,
und wobei sowohl die Alkyl- als auch die Arylgruppe gegebenenfalls bis zu sechs weitere Substituenten tragen können, die unabhängig voneinander Wasserstoff, Alkyl, O-Alkyl, OCO-Alkyl, O-Aryl, Aryl, Fluor, Chlor, Brom, lod, OH, CF₃, NO₂, NO, SiAlkyl₃, CN, COOH, CHO, SO₃H, NH₂, NH-Alkyl, N-Alkyl₂, PO-Alkyl₂, SO₂-Alkyl, SO-Alkyl, CF₃, NHCO-Alkyl, CONH₂, CO-Alkyl, COO-Alkyl, NHCOH, NHCOO-Alkyl, CO-Aryl, COO-Aryl, POAryl₂, PO₃H₂, PO(O-Alkyl)₂, SO₃-Alkyl sein können, wobei Alkyl und Aryl die oben genannte Bedeutung haben,
**dadurch gekennzeichnet, daß** in einem Lösungsmittel ein enantiomerenangereichertes Gemisch oder eine enantiomerenreine Verbindung der Formel (I), in Gegenwart eines Übergangsmetallsalzes, Übergangsmetallkomplexes oder Übergangsmetallkompexsalzes oder eines Gemisches davon, enthaltend mindestens ein Element aus der Gruppe Fe, Ru, Os, Co, Rh, Ir, Ni, Pd, Pt, umgesetzt wird.

2. Verfahren nach Anspruch 1 **dadurch gekennzeichnet, daß** Salze und/oder Komplexe der Übergangsmetalle Fe, Ru, Ir, Rh, Co und/oder Pd als Racemisierungskatalysator eingesetzt werden.

3. Verfahren nach Anspruch 1 oder 2 **dadurch gekennzeichnet, daß** der Lösung stickstoff- und/oder phosphorhaltige Liganden zugesetzt werden.

4. Verfahren nach einem Ansprüche 1 bis 3 **dadurch gekennzeichnet, daß** der Lösung Amin- und/oder Phosphanliganden zugesetzt werden.

5. Verfahren nach einem der Ansprüche 1 bis 4, **dadurch gekennzeichnet, daß** der Lösung Liganden aus der Gruppe Triphenylphosphan, Tricyclohexylphosphan, Tritert.butylphosphan, TPPTS (Tris(3-sulfonatophenyl)phosphan), Tri-n-butylphosphan, Bisdiphenylphosphinopropan, Tri-*o*-tolylphosphan, Trimethylphosphat, Diphenyl-2-pydridylphosphan, Bis(dicyclohexylphosphino)butan, Triethylamin, Pyridin, Bipyridin, Tetramethylethylendianin, Triazacyclononan oder Piperazin zugesetzt werden.

6. Verfahren nach einem der vorherigen Ansprüche **dadurch gekennzeichnet, daß** die Reaktion in Wasser und/oder in einem organischen Lösungsmittel, wie z. B. Acetonitril, Butyronitril, Essigester, Dimethoxyethan, Aceton, THF, Dioxan, Hexan, tert.-Butylmethylether und tert.-Butanol, Toluol, DMSO, Chloroform, oder einer Mischung davon, durchgeführt wird.

7. Verfahren nach einem der vorherigen Ansprüche **dadurch gekennzeichnet, daß** der Anteil an *N*-Acylaminosäure in der Lösung bei 5 - 50 % liegt.

8. Verfahren nach einem der vorherigen Ansprüche **dadurch gekennzeichnet, daß** der Racemisierungskatalysator in katalytischen Mengen zwischen 0.2 und 0.0001 Equivalenten bezogen auf die *N*-Acylaminosäure eingesetzt wird.

9. Verfahren nach einem der vorherigen Ansprüche **dadurch gekennzeichnet, daß** der Racemisierungskatalysator in katalytischen Mengen zwischen 0.1 bis 0.001 Equivalenten bezogen auf die *N*-*Ac*ylaminosäure eingesetzt werden.

10. , Verfahren nach einem der vorherigen Ansprüche **dadurch gekennzeichnet, daß** die Reaktion bei einer Temperatur von 10 - 150 °C durchgeführt wird.

11. Verfahren nach einem der vorherigen Ansprüche **dadurch gekennzeichnet, daß** die Reaktion bei einer Temperatur zwischen 40 und 80 °C durchgeführt wird.

12. Verwendung eines Verfahrens gemäß Anspruch 1 in Kombination mit einer enantioselektiven Kristallisation mit einem chiralen Racematspaltungsreagenz oder einer enantioselektiven Deacylierung mit einer Acylase zur dynamischkinetischen Racematspaltung von *N-Ac*ylaminosäuren.
